# EUROPÄISCHE PATENTANMELDUNG

(11) **EP 2 145 683 A1**
(43) Veröffentlichungstag der Anmeldung: **20.01.2010**
(21) Anmeldenummer: 08160317.7
(22) Anmeldetag: 14.07.2008
(51) Int. Cl.: B01L 3/00

(54) **Analytisches Testelement mit hydrophil modifizierter Oberfläche**

(71) Anmelder: F.HOFFMANN-LA ROCHE AG, 4070 Basel (CH); Roche Diagnostics GmbH, 68305 Mannheim (DE)
(72) Erfinder: Fürst, Otto, 68519 Viernheim (DE); Haar, Hans-Peter, 69168 Wiesloch (DE)
(74) Vertreter: Pfiz, Thomas

(57) **Zusammenfassung**

Die vorliegende Erfindung betrifft ein analytisches Testelement (10) mit einer als chemische Nachweisschicht ausgebildeten Oberfläche (14), auf der ein Spreitnetz (16) angeordnet ist. Erfindungsgemäß ist vorgesehen, dass das Spreitnetz (16) zumindest an seiner der Nachweisschicht zugewandten Oberfläche eine oxidierbare metallische Schicht (20) aufweist, die mittels eines sauerstoffhaltigen oder schwefelhaltigen Oxidationsmittels oxidiert ist.

## Beschreibung

Die vorliegende Erfindung betrifft analytische Testelemente mit hydrophil modifizierten Oberflächen.

Analytische Testelemente werden insbesondere zur schnellen qualitativen und quantitativen analytischen Bestimmung von Bestandteilen flüssiger Proben verwendet, beispielsweise in Form von vereinzelten Teststreifen oder bandförmigem Testmaterial (vgl. EP 1 039 298 B1, EP 1 593 434 A2) oder in integrierten Systemen, in denen das Testelement mit einer Probeentnahmevorrichtung verbunden ist. Die Oberfläche des Testelements ist als Nachweisschicht mit für die gewünschte Analytik geeigneten Trockenchemikalien präpariert. Wichtige Anwendungsgebiete sind beispielsweise die medizinische Diagnostik und die Umweltanalytik.

Herkömmliche Testelemente werden aus Gründen der vereinfachten und kostengünstigeren Herstellung und der Bauteilstabilität in der Regel aus Kunststoff gefertigt. Sie weisen daher allerdings eine vergleichsweise hydrophobe Oberfläche auf. Die wässrige Probe sollte sich aber an der Oberfläche verteilen und gut haften. Zu diesem Zweck wird die Oberfläche mit einem Spreitmittel versehen, beispielsweise in Form von mit einem Netzmittel beschichteten Spreitnetzen oder einer Beschichtung aus einem Netzmittel, einem Absorptions- oder einem Adsorptionsmittel.

Die Verwendung von Spreitnetzen insbesondere für Teststreifen ist an sich bekannt. In der Regel handelt es sich um Gewebe, Gewirke, etc. aus Kunststofffäden, die zur Hydrophilierung mit einer Tensid-Beschichtung versehen werden. Hierfür werden in der Regel anionische oder neutrale Tenside wie beispielsweise DONS (Docusat-Natrium) verwendet. Die Qualität dieser Beschichtungen ist allerdings Schwankungen unterworfen und umso schwieriger zu verwirklichen, je feiner die Materialstruktur ist. Insbesondere treten infolge kapillarer Trocknungseffekte eines gelösten Tensids in einem Netz Häufungs- und Verarmungszonen auf. Außerdem neigen eine Reihe von Tensiden zum Kriechen.

In der EP 1 037 717 B1 wird ferner vorgeschlagen, Kunststoffoberflächen durch flächiges Beschichten mit einem metallischen Werkstoff und anschließendes Oxidieren des Werkstoffs mit Wasser zu hydrophilieren.

Die Aufgabe der vorliegenden Erfindung besteht darin, gattungsgemäße Testelemente bzw. Filamentstrukturen mit hydrophil modifizierter Oberfläche bereitzustellen, die mit möglichst geringem Aufwand und in reproduzierbarer Qualität herstellbar sind.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Patentansprüchen.

Der Begriff "Spreitnetz" wird im Rahmen der vorliegenden Erfindung ausdrücklich als Gattungsbegriff für alle zu Spreit- bzw. Verteil- oder Transferzwecken geeignete Filamentstrukturen verstanden. Darin eingeschlossen sind u.a. Gewebe, Gewirke, Gestricke und Vliese. Der Begriff "Filament" umfasst sowohl Mono- als auch Poly-Filamente einheitlicher oder nicht-einheitlicher Materialbasis und Dimensionierung.

Unter "analytischem Testelement" werden im Rahmen der vorläufigen Erfindung alle trägergebundenen Tests für medizinische und nichtmedizinische Zwecke verstanden. Bei diesen trägergebundenen Tests sind Nachweisreagenzien in entsprechenden Schichten eines Trägers eingebettet, der mit einer flüssigen Probe in Kontakt gebracht wird. Die Reaktion von flüssiger Probe und Reagenzien führt bei Anwesenheit eines Zielanalyten zu einem nachweisbaren Signal, beispielsweise einem messbaren elektrischen Signal oder einem Farbumschlag, welcher visuell oder mit Hilfe eines Geräts beispielsweise reflexionsphotometrisch oder fluoreszenz-photometrisch ausgewertet werden kann.

Das erfindungsgemäß vorgesehene Spreitnetz liegt auf der chemischen Nachweisschicht des Testelements auf. Die aufgetragene Probenflüssigkeit wird also von dem erfindungsgemäß vorgesehenen Spreitnetz über Kapillarwirkungen zur chemischen Nachweisschicht geleitet und an den Kontaktstellen von Spreitnetz und Nachweisschicht ebenfalls durch Kapillarkräfte auf der Nachweisschicht gespreitet bzw. verteilt.

Die für die Testelemente erfindungsgemäß vorgesehenen, separat hydrophilierten Spreitnetze sind einfach herzustellen und in alle bestehenden Testsysteme (beispielsweise Teststreifen, integrierte Testsysteme) problemlos einzubringen. Sie können insbesondere problemlos verklebt werden, auch einzeln, ohne dass die hydrophilierende Beschichtung stört. Die Hydrophilierung kann in reproduzierbarer Qualität erzielt und einfach gesteuert werden. Dabei kann auf Tenside ganz verzichtet werden. Es können Testelemente mit sehr fein strukturierten Spreitnetzen hergestellt werden.

Die erfindungsgemäß vorgesehenen Spreitnetze können aus metallischen oder Kunststofffilamenten oder Kombinationen hiervon hergestellt sein. Kunststofffilamente oder daraus hergestellte fertige Spreitnetze sind in an sich bekannter Weise mit einer metallischen Beschichtung versehen, beispielsweise durch Sputtern, Metallbedampfung, galvanische Beschichtung oder Abscheiden aus gelösten Metallverbindungen. Es kann jeder metallhaltige Werkstoff verwendet werden, also reine Metalle, Legierungen und metallhaltige Mischungen. Ferner können eine oder mehrere metallische Schichten aufgetragen sein. Für die erfindungsgemäß vorgesehenen Spreitnetze eignen sich auch bekannte, käuflich erwerbbare Spreitnetze aus Kunststoff, die wie beschrieben behandelt werden.

Die Oxidation kann insbesondere mit Wasser, Alkali- oder Erdalkalihydroxiden, Sauerstoff, Wasserstoffperoxid, Ozon, Wärme in Anwesenheit von Luftsauerstoff oder Schwefelverbindungen erfolgen. Der metallische Werkstoff wird zumindest an seiner Oberfläche aufoxidiert (beispielsweise durch das Böhmit-Verfahren mit heißem Wasser oder Wasserdampf). Eine Oxidation des metallischen Werkstoffs kann auch direkt durch schwefelhaltige Verbindungen erfolgen. Ferner können die generierten sauerstoffhaltigen Metallverbindungen mit schwefelhaltigen Verbindungen nachbehandelt und ganz oder teilweise in schwefelhaltige Metallverbindungen umgewandelt werden.

Die fertige Beschichtung (im Folgenden vereinfacht als "MeO-Schicht" bzw. "MeS-Schicht" bezeichnet) entsteht als definierte Schicht aus der homogenen metallhaltigen Schicht. Es wird keine Bildung von Häufungs- und Verarmungszonen beobachtet. Die fertige MeO-Schicht bzw. MeS-Schicht weist an ihrer Oberfläche in chemischer Bindung Sauerstoff, Hydroxylgruppen bzw. anstelle von Sauerstoff ganz oder teilweise Schwefel und/oder dünnste Schichten adsorbierten Wassers auf, wodurch die Hydrophilie mit bewirkt wird. Die fertige MeO-Schicht bzw. MeS-Schicht haftet fest auf den Filamenten. Es werden keine Unterschiede bei der Beschichtungsdicke und Migration beobachtet, wie sie bei herkömmlichen Tensidbeschichtungen beispielsweise durch Trocknungs- oder Kriecheffekte auftreten können. Dadurch ist das Benetzungsverhalten auch bei sehr fein strukturierten, filigranen Spreitnetzen in Bezug auf die aufgetragene Probe reproduzierbar stabil. Wenn nur die Oberfläche der metallischen Schicht beschichtet wird und darunter metallischer Werkstoff erhalten bleibt (sei es als Metallschicht unterhalb der MeO- bzw. MeS-Schicht oder als metallischer Faden), bleiben dessen Leitfähigkeit und die Möglichkeit zu elektrostatischer Aufladung bzw. Entladung erhalten. Diese Eigenschaften können für das erfindungsgemäß vorgesehene Spreitnetz genutzt werden, beispielsweise zur Weiterverarbeitung bzw. als Fixierhilfe zur Fixierung auf dem Testelement, zur gezielten elektrostatischen Beaufschlagung des Spreitnetzes, um den Übergang bzw. den Transport der Probe auf das Spreitnetz zu vereinfachen oder zu beschleunigen.

Eine elektrostatische Aufladung kann aber auch gezielt vermieden oder abgeleitet werden. Die erfindungsgemäß vorgesehenen Spreitnetze sind gegenüber dem Stand der Technik antistatischer. Sie sind sogar gezielt elektrostatisch gesteuert einsetzbar, wodurch der Flüssigkeitstransport und/oder der Übergang zum Testelement verbessert werden. Auch die Verunreinigung durch (produktionsbedingten) Abrieb oder Fremdstaub wird reduziert oder gar vermieden.

Bei Verwendung mehrerer Metallschichten können diese so gewählt werden, dass nach deren Umwandlung zu MeO/MeS in der obersten Schicht µ-Risse auftreten, die tiefere(n) Schichte(en) jedoch kompakt bleiben. Dies resultiert in einer hydrophilen segmentierten Oberfläche mit zusätzlich verbesserter Hydrophilie bei der Aufnahme der Probenflüssigkeit. Denkbar wäre eine erste Schicht aus Silizium und eine zweite, oberste Schicht aus Aluminium vorzusehen und wie beschrieben nachzubehandeln. Die resultierende Si-O-Schicht kann mehr Wasser aufnehmen als die resultierende Al-O-Schicht, so dass diese über der Si-O-Schicht reißt.

Die Hydrophilie der Oberfläche des erfindungsgemäß vorgesehenen Spreitnetzes kann auch dadurch erhöht werden, dass bei der Herstellung der metallischen Schichten vorab eine definierte µ-Rauigkeit eingestellt wird oder µ-Partikel gezielt eingebracht werden. So wäre es denkbar, dass MeO-Partikel (beispielsweise ZnO, TiO oder ZrO₂) in den Kunststoff der Filamente eingearbeitet sind und später freigelegt werden.

Die erfindungsgemäß vorgesehenen Spreitnetze lassen sich besonders einfach in vorhandene oder neue Testsysteme integrieren. Insbesondere das Verkleben mit üblichen mehrschichtigen Teststreifen ist vor allem in miniaturisierten Systemen sicherer als bei den bisher verwendeten tensidbeschichteten Spreitnetzen.

Die räumliche Verteilung der Hydrophilie des erfindungsgemäßen Spreitnetzes lässt sich gezielt steuern, um den Transport der Probenflüssigkeit von der Oberfläche des Spreitnetzes zur Nachweisschicht des Testelements zu optimieren. Beispielsweise findet bei einem Gewebe oder Netz an dessen Kreuzungspunkten kein oder kein ausreichender Metallauftrag statt, so dass dort wegen fehlender Umwandlungen zu MeO/MeS keine Hydrophilierung erfolgt. Ferner kann das Spreitnetz selektiv abgedeckt werden, um einen Metallauftrag zu verhindern und gezielt nur selektive Bereiche des Spreitnetzes zu hydrophilieren, beispielsweise die später der Nachweisschicht des Testelements zugewandte Fläche des Spreitnetzes. Dadurch wird die Aufnahme und Speicherung der Probenflüssigkeit in für die Zwecke der Spreitung unbedeutenden Positionen des Spreitnetzes reduziert, und der Transport der Probenflüssigkeit in Richtung der Nachweisschicht wird gezielt verbessert und beschleunigt, und es geht weniger Probenflüssigkeit verloren.

Ferner kann die Geometrie der hydrophilierten Schicht der Geometrie der Nachweisschicht angepasst werden. Die räumliche Verteilung der Hydrophilie kann durch die entsprechende räumliche Verteilung der metallischen Schicht oder durch die entsprechende räumliche Verteilung des nachbehandelten Bereichs erzeugt werden. Die erzeugten hydrophilen Bereiche lassen sich durch ihre physikalischen Eigenschaften (Absorption oder Reflexion von Licht) unterscheiden. Die Unterschiede können produktionstechnisch und qualitätssichernd verwertet sowie ggf. chemisch verstärkt werden, beispielsweise durch Einfärben.

Die Hydrophilie der Oberfläche des erfindungsgemäß vorgesehenen Spreitnetzes kann durch eine zusätzliche Beschichtung mit einem Netzmittel bzw. Hydrophilierungsmittel weiter modifiziert werden (in der Regel eine Verbesserung bzw. Homogenisierung/Egalisierung; unter Umständen aber auch eine Abschwächung der Hydrophilie). Dabei kann auch eine lokale Verteilung einer erweiterten bzw. verbesserten Hydrophilie erreicht werden, indem kleinste Tröpfchen von zusätzlichen Netzmitteln lokal appliziert werden.

Bei den Hydrophilierungsmitteln handelt es sich zunächst um Netzmittel oder Dispergier-Additive im weitesten Sinne, die kationisch, bevorzugt aber anionisch oder nicht-ionisch sind und die als Reinsubstanz bei Umgebungstemperaturen bisher überwiegend in fester Form, nun aber auch flüssig eingesetzt werden können. In gewissen Anwendungen ist die flüssige Reinsubstanz auf einer adsorbierenden MeO-Schicht sogar vorzuziehen. Gerade bisher nicht übliche Hydrophilierungsmittel wie das nichtionische, flüssige Tensid Polysorbat 20 können ebenfalls in Verbindung mit einer MeO- bzw. MeS-Schicht eingesetzt werden. Diese Tenside können im Gegensatz zu den im Stand der Technik bekannten Maßnahmen zum Einsatz kommen, ohne dass unerwünschte Nebenwirkungen wie Kriechen oder Bildung von Häufungs- und Verarmungszonen auftreten. Dies liegt daran, dass die bereits hydrophile MeO- bzw. MeS-Oberflächenschicht bezüglich des Tensids adsorbierend wirkt. Daher kann die MeO- bzw. MeS-Oberflächenschicht auch direkt mit einem flüssigen nichtionischen Tensid beschichtet werden, wobei der Beschichtungs- bzw. Benetzungsvorgang aufgrund des Adsorptionsvorgangs schneller als bei herkömmlichen Kunststoff-Spreitnetzen abläuft.

Aufgrund der Hydrophilie bzw. der Adsorptionsfähigkeit der MeO- bzw. MeS-Oberflächenschicht können auch andere Netzmittel verwendet werden, die im Stand der Technik für die Beschichtung herkömmlicher Spreitnetze bisher nicht oder nur unter erheblichem Aufwand bzw. nur für beschränkte Anwendungen einsetzbar waren, weil sie für die herkömmlichen Kunststoff-Spreitnetze mit ihren hydrophoben Oberflächen nicht geeignet sind. Es handelt sich hier um solche Hydophilierungsmittel, die beim Auftragen aus wässriger Lösung für diese nicht benetzend wirken und daher auch nicht auf hydrophobe Oberflächen aufziehen. Vielmehr wirken sie erst (co-)netzend, wenn sie - auf der Oberfläche bereits aufgetragen - mit wässrigen Proben in Kontakt kommen. Dazu zählen beispielsweise sehr polare, nur wasserlösliche Hydrophilierungsmittel, die auf die erfindungsgemäß behandelten Spreitnetze nunmehr direkt aus wässriger Lösung heraus aufgetragen werden können, wie etwa die Salze von Polysäuren mit spreitender Wirkung. Bei diesen Hydrophilierungsmitteln handelt es sich um ionische Verbindungen, die zur Verdünnung in gelöster Form auf die Erstschicht (erste hydrophile Schicht) während deren Bildung oder danach aufgebracht werden, sich dabei in die Erstschicht einlagern oder damit reagieren und daher dauerhaft auf dieser haften.

So können also komplexere organische Säuren oder Salze von organischen Säuren, z.B. von Polysäuren, eingesetzt werden, die entweder synthetischer Natur oder biologischen Ursprungs sind. Beispielsweise dies sind Polycarboxylsäuren wie Polyacrylsäure oder Polymethacrylate oder Salze (z.B. Lithium- oder Natriumsalze) sulfatierter Polysaccharide wie z.B. von Heparinen oder Chondroitinsulfat oder Hyaluronsäure.

Ferner kann es sich bei ionischen Hydrophilierungsmitteln auch um Lösungen einfacher organischer Verbindungen oder Salze organischer Verbindungen handeln, wie einfache Carbonsäuren, die der Unbedenklichkeit wegen bevorzugt der Natur entstammen. So sind z. B. mono- oder oligo-Carbonsäuren oder auch Hydroxycarbonsäuren zu nennen, z.B. Bernsteinsäure oder Gluconsäure oder auch Milchsäure, Äpfelsäure, Weinsäure, Zitronensäure oder Zuckersäure bzw. deren Salze, z.B. deren Natrium-, Kalium- oder Calcium-Salze. Die Beschichtung im Falle von Salzen erfolgt bevorzugt einstufig, kann aber auch zweistufig über einen Erstauftrag von Säure und dann eine Neutralisierung mit Lauge geschehen.

Schließlich können an den funktionellen, Sauerstoff bzw. Schwefel enthaltenden Gruppen der erfindungsgemäß vorgesehenen MeO- bzw. MeS-Schichten weitere Moleküle chemisch fixiert werden, beispielsweise Nukleinsäuren oder Proteine (Enzyme, Antikörper, etc.) oder synthetische Netzmittel oder biologisch aktive Moleküle (beispielsweise gerinnungshemmende oder gerinnungsauslösende Substanzen). Auch eine Fixierung von Substanzen, die eine Benetzung mit Flüssigkeit beispielsweise durch Veränderung der Lichtabsorption oder durch Fluoreszenz indizieren können, ist denkbar.

Da die mit der erfindungsgemäß vorgesehenen MeO-Schicht bzw. MeS-Schicht modifizierte Oberfläche der erfindungsgemäß vorgesehenen Spreitnetze adsorptive bzw. reaktive Eigenschaften aufweist, kann diese außer mit Hydrophilierungsmitteln auch mit anderen Stoffen nachbehandelt und somit in ihren Eigenschaften gezielt verändert werden. Die erfindungsgemäß vorgesehene MeO-Schicht bzw. Me-S-Schicht hat beispielsweise feuchteregulierende Eigenschaften, da sie Wasser bzw. Luftfeuchte bis zu einem gewissen Grad adsorbiert. Dies verbessert die Feuchteregulierung insbesondere in einzeln verpackten Testsystemen.

Insgesamt kann also auch bei Verwendung eines feinen Spreitnetzes ein größeres Probenvolumen pro Flächeneinheit als bisher aufgenommen werden. Damit geht weniger Probenflüssigkeit verloren, und die Detektion der jeweiligen Messgröße gelingt auch bei der Verwendung besonders feiner Spreitnetze gut.

Es können lokal hydrophile Bereiche hergestellt werden. Bei der Verarbeitung und anschließenden Konfektion von vorgefertigter Flächenware, d.h. großflächigen Materialien, die nach der Behandlung zurechtgeschnitten werden, können die lokal hergestellten hydrophilen Bereiche gezielt aus dem Flächenmaterial herausgetrennt und gezielt auf der chemischen Nachweisschicht positioniert werden. Dabei kann auch ein hydrophober Umgebungsbereich mit herausgetrennt werden, so dass die Probenflüssigkeit nur über den hydrophilen Bereich des Spreitnetzes spreitet. Damit wird weniger Probenflüssigkeit benötigt und die Detektion der jeweiligen Messgröße vereinfacht.

Die Hydrophilie der Oberfläche der erfindungsgemäß vorgesehenen Spreitnetze kann auch zu weiteren Modifikationen der Oberfläche mit kapillaren Leitelementen genutzt werden, die zu weiteren vorteilhaften Eigenschaften der erfindungsgemäßen Testelemente führen.

Die hydrophile Oberfläche kann beispielsweise als Trägermaterial zur Bindung von ungerichtet oder gerichtet filternden Materialien dienen. Diese Materialien können auch einen anderen Aufbau als das Spreitnetz selbst haben. Das erfindungsgemäß vorgesehene Spreitnetz mit seiner zumindest teilweise hydrophilen Oberfläche kann beispielsweise als Träger für, auch feine und feinste, hydrophile Fasern dienen. Daraus ergibt sich eine Art Vlies und/oder eine zumindest teilweise von der Oberfläche des erfindungsgemäßen Testelements weg führende Kontaktierungsschicht in Richtung der zu applizierenden Probe. Hierfür sind sowohl Kunstfasern als auch Naturfasern, beispielsweise aus Zellulose, geeignet. Es können feinste Fasern mit einer Dicke von weniger als 50 µm und einer Länge von 10 bis 500 µm verwendet werden.

Die hydrophilen, teilweise vom Spreitnetz abstehenden Fasern wirken als kapillare Leitelemente für die zu applizierende Probe. Sie fördern den Übergang der Probe von der Probenentnahmevorrichtung auf das Spreitnetz (sog. "Durchschaltung"). Mit Beginn des Probenübergangs wird dann auch die restliche Probe über das Spreitnetz zur Nachweisschicht des erfindungsgemäßen Testelements hin verteilt. Dabei kann das Spreitnetz mit der Transportrichtung der Probenentnahmevorrichtung alle Winkel von +0° über 90° bis hin zu 180° einschließen, wobei die 90°-Orientierung bevorzugt ist.

Auf die zumindest teilweise hydrophile Oberfläche des erfindungsgemäß vorgesehenen Spreitnetzes kann eine symmetrische oder asymmetrische hydrophile Membran aufgebracht sein, die ebenfalls als kapillares Leitelement wirkt. Dabei trägt das Spreitnetz die Membran an seiner Applikationsseite für die Probe, während die gegenüberliegende die übertragende und spreitende Funktion in Richtung der Nachweisschicht erfüllt.

Eine symmetrische oder asymmetrische hydrophile Membran kann auch in das erfindungsgemäß vorgesehene Spreitnetz eingebettet sein. Hierzu ist ein Membranmaterial in die Zwischenräume des Spreitnetzes eingebracht und an die erfindungsgemäß hydrophilen Bereiche der Oberfläche des Spreitnetzes gebunden. Auch diese Anordnung wirkt als kapillares Leitelement wie oben beschrieben.

Das erfindungsgemäß vorgesehene Spreitnetz kann selbstverständlich auch nur teilweise bzw. lokal mit einem kapillaren Leitelement versehen sein und nur in gezielt ausgewählten Bereichen des Spreitnetzes ihre Wirkung entfalten, um den Transport der Probe von der Probenentnahmevorrichtung zur Nachweisschicht des Testelements weiter zu optimieren.

Die vorliegende Erfindung wird im Folgenden näher erläutert. Die einzige Figur zeigt ein analytisches Testelement in einer schaubildlichen Darstellung.

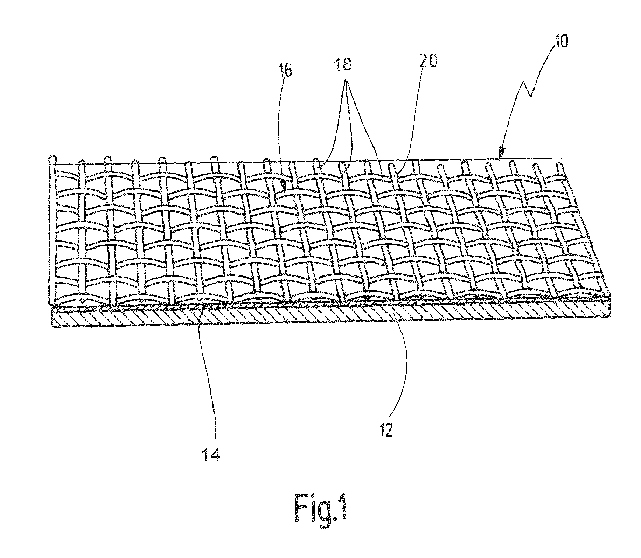

Das in Fig. 1 gezeigte Testelement 10 kann auf einem einzelnen Teststreifen oder in großer Anzahl in Abständen auf einem aufwickelbaren Testband aufgebracht sein. Das Testelement 10 weist eine Trägerfolie 12 auf, die einseitig mit einer chemischen Nachweisschicht 14 versehen ist. Die Nachweisschicht 14 besteht aus einem trockenchemischen System, das beim Aufbringen einer Probe beispielsweise durch einen Farbumschlag auf eine Zielsubstanz bzw. einen Analyten anspricht. Der Farbumschlag kann durch die transparente Trägerfolie 12 hindurch reflexionsphotometrisch erfasst werden.

Auf der von der Trägerfolie 12 abgewandten Seite der Nachweisschicht 14 ist ein Spreitnetz 16 angeordnet, das eine flächige Probenverteilung auf der Nachweisschicht 14 unterstützt. Die Probe wird beispielsweise als Blutstropfen auf der freien Seite des Spreitnetzes 16 aufgebracht. Das Spreitnetz 16 wird durch Filamente 18 gebildet, die mit einer Maschenweite von weniger als 300 µm, bevorzugt weniger als 150 µm und besonders bevorzugt weniger als 80 µm in Form eines Gewebenetzes miteinander verwoben sind. Die beispielsweise aus PET oder PA bestehenden Filamente 18 sind mit einer aus einer Metallbeschichtung gebildeten hydrophilen MeO/MeS-Oberflächenschicht 20 versehen, um eine großflächige Probenverteilung auf der Nachweisschicht 14 zu unterstützen. Die Beschichtung mit Metall kann an den Kunststoff-Filamenten als Ausgangsmaterial oder an den daraus gebildeten Gewebenetzen vorgenommen werden.

Zur Beschichtung sind insbesondere alle Metalle geeignet, die sich durch Metallbedampfung, Sputtern oder galvanische Abscheidung auftragen lassen, da diese Beschichtungsverfahren besonders einfach zu realisieren sind. Auch eine Abscheidung aus einer auf die Filamente bzw. fertigen Strukturen (Gewebe, Gestricke, Gewirke) aufgetragenen gelösten Metallverbindung ist denkbar.

Die metallische Schicht (oder metallische Filamente selbst) wird anschließend durch Nachbehandlung aufoxidiert, beispielsweise mit Wasser, Alkali- bzw. Erdalkalihydroxiden, Sauerstoff (auch Luftsauerstoff) unter Wärmebehandlung. Die metallische Schicht wird dann zumindest an ihrer Oberfläche, ggf. auch durchgehend, in ein oder mehrere Metalloxide Me₍ₓ₎O_{(y)}, Metallhydroxide Me₍ₓ₎(OH)_{(2y)} oder Mischformen daraus, wie Metalloxihydroxide (Metalloxihydrate) Me₍ₓ₎O_{(y-z)}(OH)_{(2z)}×nH₂O umgewandelt. Zur vereinfachten Darstellung werden alle diese Schichttypen unabhängig von ihrem Aufbau pauschal als "MeO-Schichten" bezeichnet.

Die MeO-Schichten weisen vorteilhafterweise eine kompakte Struktur auf und sind unlöslich oder schwerlöslich in wässrigen oder wässrig/alkoholischen Systemen. Diese Struktur ist durch die Chemie der Metallverbindungen, d.h. durch die geeignete Auswahl der Metalle bzw. Legierungen gewährleistet.

Besonders geeignet sind Zn oder Al als Metall oder Legierung. Beide reagieren leicht, wobei insbesondere Zn als Spurenelement physiologisch völlig unproblematisch ist. Mit Aluminium oder Zink oder diese Metalle enthaltenden Legierungen durch Metallbedampfung oder Sputtern beschichtete Kunststoffnetze können mit heißem Wasser oder Wasserdampf, Alkali- bzw. Erdalkalihydroxiden oder einfach durch Wärmebehandlung unter Einwirkung von Sauerstoff nachbehandelt werden, so dass zumindest die Oberfläche der Metallbeschichtung aufoxidiert wird.

Für den Bereich der Medizin, insbesondere der medizinischen Diagnostik ist die Verwendung solcher Metalle zweckmäßig, die zumindest in kleinen Mengen nicht toxisch sowie gut verträglich sind und vorzugsweise sogar Bestandteile des Körpers (auch essentielle Spurenelemente) darstellen. Beispiele hierfür sind Magnesium, Calcium, Mangan, Vanadium, Silizium und insbesondere Zink. Ebenfalls gut bis sehr gut verträglich sind Titan, Zirkonium, Silber, Aluminium, Tantal, Hafnium, Niob und Mischungen oder Legierungen daraus oder mit anderen Elementen. Dabei ist das erfindungsgemäß vorgesehene Spreitnetz als Teil des Testelements, auch wenn ein direkter Kontakt mit dem menschlichen Körper nicht stattfindet, zumindest ein Verbindungsglied zum Probenentnahme- bzw. Stechelement.

Den Mischungen können die im Herstellungsprozess üblichen geringen Beimengungen anderer Metalle als akzeptierte oder sogar gewünschte Verunreinigung beigefügt sein, wie beispielsweise Hafnium oder Yttrium als Beimengung zu Zirkonium. Ferner kann bewusst eine größere Menge an Begleitmetallen im Sinne einer Legierung zugegeben sein, beispielsweise Kupfer als Zugabe zu Aluminium in Form der beschriebenen unlöslichen hydrophilen Verbindung.

Die Verwendung derartiger verträglicher Metalle bietet sich insbesondere bei analytischen Testelementen an, wie sie beispielsweise in der EP 1 039 298 B1 beschrieben sind. Gleiches gilt für hoch integrierte Testsysteme, insbesondere im Bereich der medizinischen Diagnostik, bei denen das Testelement an eine Probenentnahmevorrichtung, beispielsweise ein Stechelement zur Blutentnahme, direkt angekoppelt ist. Bevorzugt wird hierbei Zink eingesetzt, das ein essentielles Spurenelement darstellt, wobei beispielsweise bei Diabetikern der Tagesbedarf erhöht ist. Es ist auch bekannt, Zink in Form von Zinkacetat ohne medizinische Indikation an Gesunde abzugeben, beispielsweise in Form von Geschmacksverstärkern in Kaugummis. Daher kann praktisch ausgeschlossen werden, dass eine Kontamination mit Zink gesundheitsbedenklich ist, zumal der empfohlene Tagesbedarf gegenüber einer Kontamination um Zehnerpotenzen höher liegt. Für medizinische Anwendungbereiche genauso unproblematisch, wenn auch kein essentielles Spurenelement, ist Aluminium.

Bei hoch integrierten Testsystemen ist es ferner möglich, die Probenentnahmevorrichtung (beispielsweise eine Lanzette als Stechelement) wie üblich mit einem Tensid zu beschichten, ohne die Hydrophilie der erfindungsgemäßen MeO-Schicht im Bereich des Testelements zu beeinträchtigen. Im Falle einer Migration der Tensidbeschichtung können insbesondere nichtionische Tenside von der MeO-Schicht adsorbiert werden, ohne deren primäre Hydrophilie zu beeinträchtigen. Damit wird verhindert, dass das Tensid zur die Trockenchemikalien enthaltenden Nachweisträgerschicht des Teststreifens gelangt und dessen Eigenschaften negativ beeinflusst.

Bei der Verwendung von Geweben wird in der Regel das fertige Gewebe behandelt. Es ist aber auch denkbar, lediglich die Kett- oder Schussfäden vor dem Webvorgang erfindungsgemäß zu behandeln. Denkbar sind ferner Gewebe, Gestricke oder Gewirke, die in variablen Prozentanteilen feine Metallfäden enthalten, die besonders bei sehr feinen Strukturen stabilisierend wirken. Auch hier kann eine teilweise Behandlung ausreichen.

In Modellversuchen (proof of principle) wurde Folienmaterial aus Mylar® (Handelsname für dimensionsstabile Polyethylenterephthalat-Folien) eingesetzt. Die Mylar-Folien waren mit einer dünnen Aluminiumschicht bedampft (im Folgenden: Mylar-Alu). Die Aluminiumschicht wurde unter Einwirkung von Wasserdampf aufoxidiert (vgl. EP 1 037 717 B1, im Folgenden: Mylar-Alu-Ox).

### Beispiel 1

Mylar-Alu-Ox-Folienstreifen wurden von der Rolle abgezogen und abgeschnitten. Die Stücke wurden für 1 min in siedendes demineralisiertes Wasser (im Folgenden: VE-Wasser) getaucht, dann herausgezogen, 3-fach über einen Pinzettengriff gezogen, um das Wasser abzustreifen und an der Luft hängend getrocknet.

### Beispiel 2

Mylar-Alu-Ox-Folienstreifen wurden von der Rolle abgezogen und abgeschnitten. Die Stücke wurden bei Raumtemperatur für 20 min in 0,05 % (w/w) Lithium-Heparin in Wasser (1 Liter) getaucht, dann herausgezogen und zweimal für ca. 20 s mit VE-Wasser abgewaschen, anschließend 3-fach über einen Pinzettengriff gezogen, um das Wasser abzustreifen und an der Luft hängend getrocknet.

### Beispiel 3

Mylar-Alu-Folienmaterial wurde aus der Fläche 1,5 cm breit ausgeschnitten. Die Stücke wurden für 20 min in siedendes VE-Wasser getaucht, dann herausgezogen und sofort für 1 min in 0,05 % (w/w) Lithium-Heparin in Wasser (1 Liter) getaucht. Anschließend wurden die Stücke 3-fach über einen Pinzettengriff gezogen, um die Flüssigkeit abzustreifen und an der Luft hängend getrocknet.

Als Referenzmaterial für die folgenden Vergleiche dienten Mylar-Alu-Ox-Folienstreifen, die von der Rolle abgezogen und abgeschnitten, aber nicht nachbehandelt wurden. Zum Vergleich jedes Beispiels mit dem Referenzmaterial wurden die Stücke einer an sich bekannten, standardisierten Materialprüfung für die Mylar-Alu-Ox-Folie unterzogen. Bei dieser Materialprüfung wird je n = 10 mal die Spreitwirkung auf dem Material so bestimmt, dass das Material mit 8 µl Prüflösung benetzt und deren Ausdehnung in mm (auf 0,5 mm genau) gemessen wird. Die zehn Einzelwerte wurden addiert und daraus der Mittelwert gebildet. Der angegebene Wert stellt also eine Größe für die durchschnittliche lineare Spreitung dar. Eine noch genauere Darstellung bezüglich der benetzten Fläche wird erhalten, wenn die linearen Einzelwerte quadriert und dann erst der Mittelwert aus der Summe gebildet wird.

Die Ergebnisse sind in der folgenden Tabelle 1 zusammengefasst.

**Tabelle 1**

| Spreitung (mm) | Referenz | Beispiel 1 | Beispiel 2 | Beispiel 3 |
|---|---|---|---|---|
| 1 | 7,0 | 6,0 | 8,0 | 8,0 |
| 2 | 8,0 | 6,0 | 8,0 | 8,0 |
| 3 | 7,0 | 6,0 | 8,0 | 8,0 |
| 4 | 8,0 | 6,5 | 9,0 | 6,5 |
| 5 | 7,0 | 7,0 | 8,5 | 7,0 |
| 6 | 6,5 | 7,0 | 8,5 | 8,0 |
| 7 | 8,0 | 8,0 | 7,5 | 8,0 |
| 8 | 6,5 | 6,0 | 7,0 | 7,0 |
| 9 | 6,5 | 7,0 | 6,5 | 8,0 |
| 10 | 7,0 | 6,0 | 6,5 | 8,0 |
| **Mittelwert** | **7,2** | **6,6** | **7,8** | **7,7** |
| Soll | ≥ 6,0 mm | | | |

### Vergleich Referenz / Beispiel 1

Das Ergebnis zeigt, dass das Eintauchen der bereits hydrophilierten Mylar-Alu-Ox-Folie in heißes Wasser eine Verschlechterung der Spreitwirkung zu beobachten ist. Eine "Auffrischung" der Hydrophilierung findet also nicht statt. Daher wurden die Versuche aus den Beispielen 2 und 3 bei Raumtemperatur vorgenommen.

### Vergleich Referenz / Beispiel 2

Die zusätzliche Beschichtung mit einem Netzmittel, hier Lithium-Heparin, führt zu einer deutlichen Verbesserung der Spreitwirkung.

### Vergleich Referenz / Beispiel 3

Die Oxidation eines Mylar-Alu-Folienmaterial in heißem Wasser mit anschließender Behandlung mit einem Netzmittel (hier Lithium-Heparin) führt ebenfalls zu einer deutlichen Verbesserung der Spreitwirkung.

## Patentansprüche

1. Analytisches Testelement mit einer als chemische Nachweisschicht (14) ausgebildeten Oberfläche, auf der ein Spreitnetz (16) zum flächigen Verteilen einer Flüssigprobe angeordnet ist, **dadurch gekennzeichnet, dass** das Spreitnetz (16) zumindest an seiner der Nachweisschicht (14) zugewandten Oberfläche eine oxidierbare metallische Schicht (20) aufweist, die mittels eines sauerstoffhaltigen oder schwefelhaltigen Oxidationsmittels oxidiert ist.

2. Testelement nach Anspruch 1, **dadurch gekennzeichnet, dass** das Spreitnetz (16) metallische Filamente (18) und/oder Kunststoff-Filamente aufweist.

3. Testelement nach Anspruch 1 oder 2, **dadurch gekennzeichnet, dass** das Spreitnetz (16) eine einlagige oder mehrlagige metallische Schicht (20) aufweist.

4. Testelement nach einem der vorhergehenden Ansprüche, **dadurch gekennzeichnet, dass** die metallische Schicht (20) Aluminium oder Zink enthält.

5. Testelement nach einem der vorhergehenden Ansprüche, **dadurch gekennzeichnet, dass** die metallische Schicht (20) ein reines Metall, eine Legierung oder eine metallhaltige Mischung ist.

6. Testelement nach einem der vorhergehenden Ansprüche, **dadurch gekennzeichnet, dass** die metallische Schicht (20) mittels Wasser, Sauerstoff, Alkali-oder Erdalkalihydroxid oxidiert ist.

7. Testelement nach Anspruch 6, **dadurch gekennzeichnet, dass** die oxidierte metallische Schicht (20) mit einer schwefelhaltigen Verbindung nachbehandelt ist.

8. Testelement nach einem der vorhergehenden Ansprüche, **dadurch gekennzeichnet, dass** die metallische Schicht (20) entweder an ihrer Oberfläche oder durchgehend oxidiert ist.

9. Testelement nach einem der vorhergehenden Ansprüche, **dadurch gekennzeichnet, dass** mindestens ein kapillares Leitelement auf dem Spreitnetz (16) angeordnet und/oder in dem Spreitnetz (16) eingebettet ist.

10. Testelement nach Anspruch 9, **dadurch gekennzeichnet, dass** das mindestens eine kapillare Leitelement als hydrophiles Fasermaterial oder als hydrophile Membran ausgebildet ist.

11. Testelement nach einem der vorhergehenden Ansprüche, **dadurch gekennzeichnet, dass** das Spreitnetz (16) hydrophilierte und hydrophobe Oberflächenbereiche aufweist.

12. Testelement nach einem der vorhergehenden Ansprüche, **dadurch gekennzeichnet, dass** es als Teststreifen oder Testband ausgebildet ist oder in ein integriertes Testsystem aufgenommen ist.

13. Testelement nach einem der vorhergehenden Ansprüche, **dadurch gekennzeichnet, dass** das Spreitnetz (16) mit mindestens einem Hydrophilierungsmittel nachbehandelt ist.

14. Testelement nach Anspruch 13, **dadurch gekennzeichnet, dass** mindestens ein Hydrophilierungsmittel ein anionisches oder nichtionisches Tensid ist.

15. Testelement nach Anspruch 13 oder 14, **dadurch gekennzeichnet, dass** mindestens ein Hydrophilierungsmittel ein nichtionisches Tensid ist, welches im Temperaturbereich der Anwendung als Reinsubstanz flüssig ist.

16. Testelement nach Anspruch 13 oder 14, **dadurch gekennzeichnet, dass** mindestens ein Hydrophilierungsmittel eine zumindest weitgehend wasserlösliche Verbindung, beispielsweise eine organische Säure oder eine organische Polysäure oder deren Salz ist.

17. Filamentstruktur insbesondere in Form eines Gewebes, Gewirkes, Gestrickes oder Vlieses mit einer oxidierbaren metallischen Schicht (20), die zumindest bereichsweise mittels eines sauerstoffhaltigen oder schwefelhaltigen Oxidationsmittels oxidiert und gegebenenfalls zusätzlich mit einem Hydrophilierungsmittel versehen ist.

18. Verwendung einer Filamentstruktur nach Anspruch 17 als Spreitnetz (16) für ein analytisches Testelement (10) zum flächigen Verteilen einer Flüssigprobe.
